# EUROPÄISCHE PATENTSCHRIFT

(11) **EP 1 185 494 B1**
(45) Veröffentlichungstag und Bekanntmachung des Hinweises auf die Patenterteilung: **23.04.2003**
(21) Anmeldenummer: 01915130.7
(22) Anmeldetag: 10.01.2001
(51) Int. Cl.: C07C 51/215, C07C 57/145

(54) **VERFAHREN ZUR KATALYTISCHEN GASPHASENOXIDATION ZU MALEINSÄUREANHYDRID**
METHOD OF GAS PHASE CATALYTIC OXIDATION TO GIVE MALEIC ACID ANHYDRIDE
PROCEDE D'OXYDATION CATALYTIQUE EN PHASE GAZEUSE POUR PRODUIRE DE L'ANHYDRIDE D'ACIDE MALEIQUE

(30) Priorität: 10.01.2000 DE 10000584
(43) Veröffentlichungstag der Anmeldung: 13.03.2002
(73) Patentinhaber: BASF AKTIENGESELLSCHAFT, 67056 Ludwigshafen (DE)
(72) Erfinder: ZEHNER, Peter, 67071 Ludwigshafen (DE); MACHHAMMER, Otto, 68163 Mannheim (DE); HECHLER, Claus, 67063 Ludwigshafen (DE); WECK, Alexander, 67251 Freinsheim (DE); OLBERT, Gerhard, 69221 Dossenheim (DE); STABEL, Uwe, 67166 Otterstadt (DE)
(74) Vertreter: Isenbruck, Günter, Dr.
(86) Internationale Anmeldenummer: EP0100234
(87) Internationale Veröffentlichungsnummer: WO01051448

(56) Entgegenhaltungen:
- WO-A-01/32301
- DE-A- 2 016 614
- DE-A- 10 031 347
- DE-A- 19 857 842
- US-A- 4 544 544

## Beschreibung

Die Erfindung betrifft ein Verfahren zur Herstellung von Maleinsäureanhydrid durch heterogen katalysierte Gasphasenoxidation.

Maleinsäureanhydrid hat eine beträchtliche technische Bedeutung. So ist es sowohl zur Polykondensation als auch zur Polymerisation verwendbar, wobei Polyesterharze und Alkydharze die bedeutendsten technischen Folgeprodukte sind. Daneben ist Maleinsäureanhydrid das Ausgangsmaterial für kommerziell wichtige Chemikalien, wie zum Beispiel Bernsteinsäureanhydrid, Gamma-Butyrolacton, 1,4-Butandiol und Tetrahydrofuran.

In bekannten großtechnischen Verfahren zur Gasphasenoxidation zu Maleinsäureanhydrid wird ein n-Butan, Sauerstoff und weitere Komponenten, wie Stickstoff und Wasserdampf, enthaltendes Reaktionsgemisch in einer aus Einzelpartikeln bestehenden Katalysatorschüttung bei 320 bis 480°C zu einem Reaktionsgemisch umgesetzt, das neben der Hauptkomponente Maleinsäureanhydrid weiterhin Wasserdampf, Kohlenmonoxid, Kohlendioxid, nicht umgesetztes Butan, Inertgase, zum Beispiel Stickstoff, und weitere organische Spurenkomponenten enthält. Die dabei freiwerdende Reaktionsenthalpie heizt das Reaktionsgemisch auf. Eine starke Erwärmung des Reaktionsgemisches verringert die Ausbeute an dem gewünschten Wertprodukt Maleinsäureanhydrid durch unselektive Überoxidation, wobei bei sehr starker Erwärmung die Gefahr besteht, daß die Umsetzung in eine homogene Verbrennungsreaktion in der Gasphase mit starkem Temperatur- und Druckanstieg umschlägt.

Um den Temperaturanstieg begrenzen zu können, wird deshalb in den eingesetzten Reaktoren die Reaktionsenthalpie teilweise über die den Katalysator umgebenden Reaktorwände abgeführt. Zu diesem Zweck wird der Reaktionsraum in viele, parallel geschaltete, als Rohre mit einem Innendurchmesser von 20 bis 45 mm ausgebildete Einzelreaktionsräume aufgelöst. Die mit Katalysatorschüttung gefüllten, senkrecht stehenden Einzelrohre werden von oben nach unten mit Reaktionsgemisch durchströmt. Dabei wird mehr als 60% der Reaktionsenthalpie über die Rohrwand an ein die Rohre umströmendes Wärmetauschmittel abgegeben. Als Wärmetauschmittel werden in der Regel anorganische Salzschmelzen eingesetzt, es können jedoch auch organische Wärmetauschmittel, Metallschmelzen oder Gase wie Helium verwendet werden. Um die Erwärmung des Wärmetauschmittels im Reaktor auf kleiner als 20°C zu begrenzen, sind hohe Umwälzmengen und entsprechende Pumpenleistungen erforderlich. Die Rückkühlung des Wärmetauschfluids erfolgt meist durch Erzeugung von Wasserdampf an vom Wärmetauschmittel durch- oder umströmten Rohren.

Für eine hohe Selektivität der Umsetzung ist eine Begrenzung der axialen und radialen Temperaturgradienten im Reaktionsrohr auf kleiner als 30°C erforderlich. Dabei bleibt für den axialen Temperaturgradienten die interne Vorheizstrecke im Reaktionsrohr, in der das Reaktionsgemisch auf die Reaktionstemperatur aufgeheizt wird, und die auch in einem dem eigentlichen Reaktor vorgeschalteten Wärmetauscher gelegt werden kann, außer Betracht. Der maximal zulässige radiale Temperaturgradient legt den maximalen Rohrdurchmesser fest, während die für einen ausreichenden konvektiven Wärmeübergang im mit Katalysatorschüttung gefüllten Rohr notwendige Mindestgasgeschwindigkeit zusammen mit der spezifischen Reaktionsgeschwindigkeit die Mindestrohrlänge festlegt Um diesen Forderungen zu genügen, enthalten bei großtechnischen Verfahren mit einer Erzeugung von etwa 30.000 t/a Maleinsäureanhydrid die Reaktoren zwischen 12.000 und 40.000 parallel geschaltete Reaktionsrohren. Die Mindestrohrlänge und die Mindestgasgeschwindigkeit führen wiederum zu einem Strömungsdruckverlust über den Reaktor von mehr als 0,4 bar. Um eine ungleichmäßige Anströmung der Reaktionsrohre und damit entsprechende Selektivitätseinbußen und die Gefahr eines Umschlagens in eine homogene Verbrennungsreaktion in der Gasphase durch lokale Überhitzung zu vermeiden, wird daher der Druckverlust in jedem der zahlreichen Reaktionsrohre üblicherweise aufwendig abgeglichen. Daher ist das Befüllen des Reaktors mit Katalysator und dessen Entfernung sehr zeit- und kostenintensiv.

Derartige Reaktoren haben somit zum einen den Nachteil, daß sehr viele Einzelreaktionsrohre erforderlich sind, was zu einer aufwendigen Bauart und hohen Kosten führt. Darüber hinaus ist nachteilig, daß ein Zwischenkreislauf mit einem Wärmetauschmittel erforderlich ist, wodurch wiederum hohe Pumpenleistungen erforderlich sind und sich additive Kosten für die Rückkühlung des Wärmetauschmittels ergeben. Darüber hinaus tritt ein Exergieverlust auf. Ein weiterer Nachteil ist, daß der hohe Druckverlust in den einzelnen Reaktionsrohren und der hierfür notwendige Abgleich zu einer sehr aufwendigen Handhabung des Katalysators führen.

Die DE-C-197 54 185 beschreibt beispielsweise einen Reaktor mit einem zylinderförmigen Reaktorbehälter, wobei im Reaktorbehälter als Thermobleche ausgebildete Wärmetauscherplatten in vertikaler Orientierung auf dem Siebboden des Reaktors nebeneinander, mit vorgegebenem Abstand voneinander, angeordnet sind. Die Platten werden von einem Kühlmedium durchströmt, das im Bereich der Behälterdecke über geeignete Einrichtungen den Wärmetauscherplatten zugeführt und im Bereich des Behälterbodens über geeignete Einrichtungen aus den Wärmetauscherplatten abgeführt wird. Zwischen den Wärmetauscherplatten wird im Gegenstrom zum Kühlmedium ein gasförmiges Reaktionsmedium, mit Zuführung im Bereich des Behälterbodens und Abführung im Bereich der Behälterdecke geleitet. Die Druckschrift gibt keinerlei Hinweis darauf, daß ein derartiger Reaktor für die heterogen katalysierte Gasphasenoxidation zu Maleinsäureanhydrid eingesetzt werden kann.

Die DE-A-197 19 375 beschreibt ein Verfahren zur Herstellung von Ethylenoxid durch katalytische Gasphasenoxidation von Ethylen mit Sauerstoff in einem Reaktor, wobei der Katalysator in Reaktionszonen zwischen Wärmetauscherplatten angeordnet ist und vom gasförmigen Reaktionsgemisch durchströmt wird. Bei der katalytischen Gasphasenoxidation zu Ethylenoxid wird eine vergleichsweise geringe Wärmemenge je Volumeneinheit des Katalysators entwickelt.

Die Aufgabe der vorliegenden Erfindung bestand darin, ein Verfahren zur Herstellung von Maleinsäureanhydrid zu schaffen, das eine erhöhte Wirtschaftlichkeit, insbesondere bezüglich des Wärmetauschmittelaufwandes, auch bei sehr hohen Umsätzen sowie bei Anlagen mit großer Kapazität zeigt.

Zur Lösung dieses Problems wird vorgeschlagen, die heterogen katalysierte Gasphasenoxidation zu Maleinsäureanhydrid in einem Reaktionsraum zwischen Wärmetauscherplatten und somit einer zweidimensional, über den Reaktorquerschnitt ausgedehnten Katalysatorschüttung durchzuführen. In überraschender Weise wurde dabei eine nicht vorhersehbare Selektivitätssteigerung der Bildung von Maleinsäureanhydrid gefunden.

Somit betrifft die Erfindung ein Verfahren zur Herstellung von Maleinsäureanhydrid durch heterogen katalysierte Gasphasenoxidation in einem Reaktor mit Zuführung für das Reaktionsgemisch an einem Reaktorende und Abführung des Produktgemisches am gegenüberliegenden Reaktorende sowie mit im Reaktorinnenraum angeordneten Einrichtungen zur Abführung der Reaktionswärme, die von einem Wärmetauschmittel durchströmt sind, das dadurch gekennzeichnet ist, daß die Einrichtungen Wärmetauscherplatten sind.

Bevorzugte Ausführungsformen der Erfindung sind in der nachfolgenden Beschreibung, den Figuren und den abhängigen Ansprüchen definiert.

Als Ausgangsverbindungen können grundsätzlich alle zur Herstellung von Maleinsäureanhydrid bekannten Edukte verwendet werden, insbesondere n-Butan, n-Buten oder Gemische davon, sowie Benzol oder Butadien. Am bevorzugtesten ist die Herstellung aus n-Butan. Zweckmäßigerweise wird die katalytische Gasphasenoxidation mit molekularem Sauerstoff oder einem diesen enthaltenden Gas, z.B. Luft, durchgeführt, wobei Luft besonders bevorzugt ist. Daneben kann das Reaktionsgemisch noch weitere Komponenten, wie zum Beispiel Stickstoff, Wasserdampf oder andere inert wirkende Verdünnungsgase, wie z.B. Kohlendioxid, Kohlenmonoxid, iso-Butan und/oder Methan, enthalten.

Vorzugsweise wird die Gasphasenoxidation bei Temperaturen im Bereich von 320° bis 480°C, insbesondere 380° bis 450°C, und ggf. erhöhtem Druck, vorzugsweise bei einem Druck von 1 bis 6 bar absolut, durchgeführt.

Es können alle heterogenen Katalysatoren eingesetzt werden, die im Stand der Technik zur Herstellung von Maleinsäureanhydrid bekannt sind. Vorzugsweise werden als heterogene Katalysatoren oxidische Mehrkomponenten-Katalysatoren auf der Basis der Oxide von Vanadium und Phosphor (sog. V-P-O-Katalysatoren), zum Beispiel (VO)₂P₂O₇, eingesetzt. Diesen Katalysatoren können Promotoren, zum Beispiel Zink, Zirkonium, Bismut, Antimon, Zinn, Nickel, Kobalt, Eisen, Chrom, Mangan und/oder Molybdän, zugesetzt werden. Bei Verwendung von Benzol als Ausgangsverbindung können auch Katalysatoren auf der Basis der Oxide von Vanadium und Molybdän eingesetzt werden. Die Form des Katalysators unterliegt keiner besonderen Beschränkung. Es kann jede feste geometrische Form verwendet werden, die Einzelteilchen umfaßt und Poren enthält. Die Katalysatorteilchen können beispielsweise von zylindrischer, kubischer, konischer, prismatischer, pyramidaler oder trilobaler Form sein. Vorzugsweise besteht der Katalysator aus Einzelpartikeln mit einem hydraulischen Durchmesser im Bereich von 3 bis 8 mm. Der Katalysator kann auch in Form eines Trägerkatalysators vorliegen, so wie es beispielsweise in der DE-OS-2 351 151 beschrieben ist, bei der das katalytisch aktive Material auf einen inerten Träger aufgebracht ist. Es besteht auch die Möglichkeit, daß Katalysatoren unterschiedlicher Aktivität und/oder unterschiedlicher Form gleichzeitig eingesetzt werden, wobei zur besseren Steuerung der Eigenschaften des Katalysators dem Reaktionsgemisch auch geringe Mengen an einer Phosphorverbindung, insbesondere einer phosphororganischen Verbindung, zugefügt werden können. Die Eigenschaften des Katalysators können auch gesteuert werden, indem Katalysatorteilchen unterschiedlicher Größe oder zusätzlich inerte Feststoffmaterialien als Verdünnungsmittel verwendet werden.

Geeignete Reaktionsgemische enthalten 0,5 bis 10 Vol.-% der oben genannten Ausgangsverbindung, 6 bis 30 Vol.-% Sauerstoff und als Rest weitere Komponenten, zum Beispiel Stickstoff, Wasserdampf und/oder andere inert wirkende Verdünnungsgase, insbesondere wie sie oben definiert wurden, jeweils bezogen auf 100 Vol.-% Reaktionsgemisch.

Bei der katalytischen Gasphasenoxidation von C₄-Ausgangsverbindungen wird nicht reines Maleinsäureanhydrid, sondern ein Produktgemisch erhalten, das neben Maleinsäureanhydrid als Nebenprodukte zum Beispiel Essigsäure, Acrylsäure, Crotonsäure, Kohlenmonoxid, Kohlendioxid, nicht umgesetzte Ausgangsverbindungen, etc. enthält. Üblicherweise enthält das Produktgemisch, jeweils bezogen auf das gesamte Produktgemisch, 0,2 bis 4 Vol.-% Maleinsäureanhydrid, jeweils 0,01 bis 0,09 Vol.-% Essigsäure und/oder Acrylsäure und als Rest Stickstoff, Kohlendioxid, Kohlenmonoxid, Wasserdampf und/oder weitere inert wirkende Verdünnungsgase. Bei der Benzol-Oxidation treten in geringen Mengen als Nebenprodukte Benzochinon, Formaldehyd und Ameisensäure auf.

Die Form des Reaktors ist grundsätzlich keinen Einschränkungen unterworfen. Es können übliche zylindrische Reaktoren, jedoch auch quaderförmige Reaktoren eingesetzt werden. Ebenso bestehen keine Einschränkungen bezüglich der Ausrichtung der Reaktoren; die Reaktoren können grundsätzlich in jeder Position ausgerichtet sein, wobei für den Sonderfall der zylindrischen Reaktoren eine vertikale Ausrichtung in der Regel bevorzugt ist.

Erfindungsgemäß werden für das Verfahren zur Gasphasenoxidation zu Maleinsäureanhydrid Reaktoren mit Einrichtungen zur Abführung der Reaktionswärme eingesetzt, die als Wärmetauscherplatten ausgebildet sind. In einer bevorzugten Ausführungsform der Erfindung werden mindestens 60% der im Reaktor freiwerdenden Reaktionsenthalpie über die Wärmetauscherplatten abgeführt. Ein Teil der Reaktionsenthalpie kann auch mit dem Reaktionsgas ausgetragen werden oder im Reaktor wird ein Teil der Wärmeaustauschfläche in konventioneller Weise bereitgestellt. Hierbei kann ein Teil der Wärmeaustauschfläche auch mit Gasraum (Zwischenkühler), oder Inertmaterial (Vorwärmstrecke, Nachkühler) umgeben sein.

Wärmetauscherplatten sind überwiegend flächenförmige Gebilde, die einen mit Zu- und Abführleitungen versehenen Innenraum mit geringer Dicke im Verhältnis zur Fläche aufweisen. Sie werden in der Regel aus Blechen, häufig aus Stahlblechen, hergestellt. Je nach Anwendungsfall, insbesondere den Eigenschaften des Reaktionsmediums sowie des Wärmetauschmittels können jedoch spezielle, insbesondere korrosionsfeste, Werkstoffe zum Einsatz kommen. Die Zu- bzw. Abführeinrichtungen für das Wärmetauschmittel sind in der Regel an einander entgegengesetzten Enden der Wärmetauscherplatten angeordnet. Vorzugsweise bestehen die Wärmetauscherplatten aus mindestens zwei, am Umfang druckdicht verbundenen und in einem Raster von 15 bis 80 mm durch punktförmige Verbindung, vorzugsweise Punktschweißen, gegeneinander abgestützte und durch Innendruck aufgeweitete Metallbleche, deren so gebildeter Innenraum von dem Wärmetauschmittel durchströmt wird. In einer besonders bevorzugten Ausführungsform weisen die Wärmetauscherplatten eine Breite von 0,5 bis 3 m, eine maximale Länge von 8 m und eine Gesamtdicke von 5 bis 100 mm auf. In einer weiteren bevorzugten Ausführungsform werden die Wärmetauscherplatten aus mindestens vier Einzelblechen so aufgebaut, daß zwei die zentrale, mit Wärmetauschmittel durchströmte Tasche umgebende, vorzugsweise mit Gas gefüllte, Isoliertaschen entstehen.

Bezüglich der Anordnung der Wärmetauscherplatten im Reaktor gibt es grundsätzlich keine Einschränkungen; die Wärmetauscherplatten können beispielsweise spiralförmig, konzentrisch oder radial im Reaktor angeordnet sein. Sie können auch in das Hüllrohr des Reaktors eingehängt werden. Vorzugsweise werden sie zu planparallelen Paketen geschichtet, zu Ringen oder Ringsegmenten gebogen und dann konzentrisch angeordnet, oder spiralförmig gebogen, wobei vorzugsweise zwischen den Platten ein Zwischenraum von mindestens 4 und höchstens 60 mm verbleibt. Es besteht auch die Möglichkeit, Plattenpakete schräg zu stellen, wobei mindestens ein Paket aus Blechen so angeordnet wird, daß die von den Platten gebildeten Strömungskanäle zur Mittelachse des Reaktors einen Winkel von 0 bis 20° aufweisen. Die Oberfläche der Wärmetauscherplatten kann auch mit einem Distanzhalter bis hin zur Katalysatorschicht versehen werden, was vorzugsweise durch Aufbringen eines Streckmetallnetzes oder Inertmaterials erfolgt. Daneben können einzelne Plattenpakete bei unterschiedlichen Wärmeaustauschmitteltemperaturen betrieben werden, wobei die Temperatur längs der Reaktorachse variiert. Eine derartige Temperaturzonung ermöglicht eine Anpassung an unterschiedliche Wärmestromdichten. Daneben kann der Plattenabstand der einzelnen Plattenpakete in Strömungsrichtung entsprechend der mit zunehmendem Reaktionsfortschritt abnehmenden Leistungsdichte zunehmen, was die zuvor genannte Temperaturzonung ergänzt und/oder ersetzt.

Vorteilhaft können auch Wärmetauscherplatten eingesetzt werden, die keilförmig ausgebildet sind, d.h. deren von Wärmetauschmittel durchströmter Innenraum bevorzugt kontinuierlich in Richtung des Reaktionsgemischstromes abnimmt.

Derartige keilförmige Wärmetauscherplatten können beispielsweise hergestellt werden, indem zwei Bleche übereinandergelegt und in zunehmend größeren Abständen verschweißt werden. Die Platten werden anschließend in eine leicht geneigte Aufblasvorrichtung eingeklemmt und auf einen vorgegebenen Abstand aufgeblasen. Mittels keilförmig ausgebildeter Wärmetauscherplatten kann die Anpassung an das Temperaturprofil der Reaktion optimiert werden. In einer weiteren vorteilhaften Ausgestaltung können die Wärmetauscherplatten vollständig oder partiell längs verschweißt sein. Dazu werden jeweils zwei Bleche übereinander gelegt, durch Rollnahtschweißung über Längsnähte verschweißt und mittels einer geeigneten Aufblasvorrichtung aufgeblasen.

Gemäß einer weiteren Ausführungsvariante sind im Reaktorinnenraum und denselben im wesentlichen vollständig ausfüllend, ebene, rechteckige, parallel zueinander ausgerichtete Bleche eingebracht, wobei an jedem Blech jeweils zwei gegenüberliegende Seiten in dieselbe Richtung rechtwinklig abgekantet sind und beim jeweils darauffolgenden Blech die anderen beiden einander gegenüberliegenden Seiten in dieselbe Richtung um denselben Abstand rechteckig abgekantet sind, dergestalt, daß jeweils quaderförmige Räume entstehen, wobei die jeweils benachbarten Räume im Querstrom vom Reaktionsgemisch beziehungsweise vom Wärmetauschmittel durchströmt werden.

Gemäß einer weiteren Ausgestaltung sind die Wärmetauscherplatten in Längsrichtung des Reaktors parallel zueinander angeordnet.

In einer weiteren Variante sind Plattenpakete derart versetzt, daß die Orientierung der Plattenquerachse zwischen mindestens zwei aufeinanderfolgenden Plattenpaketen zwischen 0 und 90° abweicht.

Bezüglich der in dem erfindungsgemäßen Verfahren einsetzbaren Wärmetauschmittels gibt es grundsätzlich keine Einschränkungen. Es können sowohl anorganische wie auch organische flüssige Wärmetauschmittel eingesetzt werden, die bei der Reaktionstemperatur der katalytischen Gasphasenoxidation im flüssigen Aggregatszustand verbleiben oder teilweise oder ganz verdampfen. Beispielsweise ist eine Salzschmelze besonders geeignet. Daneben können auch gasförmige Wärmetauschmittel, vorzugsweise Helium, eingesetzt werden. Besonders vorteilhaft ist es, ein Wärmetauschmittel einzusetzen, das bei der Reaktionstemperatur der katalytischen Gasphasenoxidation ganz oder zumindestens teilweise verdampft. Besonders bevorzugt ist hierfür Wasser. Durch Ausnutzung der Siedekühlung wird hierbei eine effiziente Wärmeabführung gewährleistet, wobei für die Abführung derselben Wärmemenge gegenüber dem Einsatz eines Wärmetauschmittels, das seinen Aggregatszustand nicht ändert, eine wesentliche Einsparung der erforderlichen Menge erreicht wird. In einer bevorzugten Ausgestaltung werden dem Wärmetauschmittel zum Zwecke der Anhebung des Siedepunktes hochsiedende Stoffe, vorzugsweise mehrwertige Alkohole, zugesetzt, oder diese in reiner Form verwendet.

Siedekühlung kann sowohl im Gleich- wie auch im Gegenstrom erfolgen. Bei Betrieb im Gleichstrom mit Anströmung von unten besteht zusätzlich die Möglichkeit, den Stand der siedenden Flüssigkeit so zu regulieren, daß gegen Ende des Reaktors eine geringere Wärmeabfuhr erfolgt und durch das dort nun höhere Temperaturniveau die Gesamtausbeute erhöht wird. Bei Siedekühlung stellt sich auf der Kühlmediumseite entsprechend der Temperatur ein definierter Dampfdruck ein (bei Wasser Werte im Bereich von etwa 20 bis 160 bar), so daß eine entsprechend druckfeste Auslegung der Kühlmediumseite des Apparats erforderlich ist.

In einer weiteren bevorzugten Ausgestaltung der Erfindung werden die Wärmetauscherplatten von einem Gemisch aus Wärmetauschmitteldampf und -flüssigkeit in Form von Blasen, Tropfen und/oder einem Aerosol durchströmt. Hierbei kann gezielt soviel Wärmetauschmittel eingedüst werden, daß der Wärmeübergang weitgehend über die Gasphase erfolgt und damit gezielt verschlechtert wird.

Erfindungsgemäß wird das Reaktionsgemisch an einem Reaktorende dem Reaktorinnenraum zwischen den Wärmetauscherplatten zugeführt und am entgegengesetzten Reaktorende abgeführt. Das Reaktionsgemisch durchströmt somit den Reaktor durch den Zwischenraum zwischen den Wärmetauscherplatten. Dadurch findet eine ständige Quervermischung des Reaktionsgemisches statt mit der Folge einer hohen Homogenität desselben.

Das Wärmetauschmittel und Reaktionsgemisch können im Gleichstrom, Gegenstrom oder Kreuzstrom durch den Reaktor geführt werden. Besonders bevorzugt wird eine Gleichstromführung, da dadurch eine bessere Anpassung an das Temperaturprofil der Reaktion gewährleistet wird.

Gemäß einer weiteren bevorzugten Ausführungsform können zwei oder mehrere Reaktionszonen mit getrennten Wärmetauschmittel-Kreisläufen in Richtung des Reaktionsgemischstromes angeordnet sein.

Der Katalysator kann in den Zwischenraum zwischen den Wärmetauscherplatten eingeführt werden, beispielsweise in Form einer ungeordneten Schüttung. Das Einbringen und der Wechsel der Katalysatorschüttung ist dabei einfacher und gleichmäßiger gegenüber dem Einfüllen in die Reaktionsrohre bei bekannten Verfahren. Es entstehen größere zusammenhängende Reaktionsräume und die Verstopfungsgefahr der Katalysatorschüttung ist kleiner. Bei Anordnung des Katalysators in einer Schüttung um die Wärmetauscherplatten ist von Vorteil, daß sich das Reaktionsgas im Fall von lokalen Verlegungen nach dem Vorbeiströmen an der Verengung wieder auf den vollen Katalysatorquerschnitt zwischen zwei Wärmetauscherplatten verteilen kann und der gesamte Reaktionsquerschnitt zum Umsatz beitragen kann. Ein weiterer Vorteil der Wärmeträgerplatten ist die im Vergleich zum Rohrbündelreaktor leichtere Entleerung des Katalysators im Falle eines Katalysatorwechsels.

Es ist jedoch auch möglich, zusätzlich oder alternativ zur Katalysatorschüttung die Wärmetauscherplatten an ihren vom Reaktionsgemisch überströmten Außenseiten katalytisch zu beschichten. Dabei wird der Katalysator mit einer Schichtdicke im Bereich zwischen 0,1 und 1,5 mm direkt auf die Platten aufgebracht. Vorzugsweise werden die zu beschichtenden Platten durch mechanische Verfahren, zum Beispiel Sandstrahlen, Kugelstrahlen oder chemische Verfahren, zum Beispiel Anätzen, Vorbeschichten, vorbehandelt. Wegen der im wesentlichen ebenen Form der Wärmetauscherplatten können diese im Vergleich zu Reaktionsrohren einfacher beschichtet werden.

Somit umfaßt das erfindungsgemäße Verfahren die folgenden Merkmale und Vorteile.
- Einfache Bauart mit austauschbaren Wärmetauscherplatten; bei Verwendung standardisierter Bleche für die Platten können weitere Kosten eingespart werden;
- eine hohe Druckfestigkeit ermöglicht eine effektive Kühlung unter Erzeugung von Direktdampf, wobei kein Zwischenkreislauf erforderlich ist; damit geringer spezifischer Wärmetauschmitteldurchsatz und somit geringere Pumpenleistung und verringerter Exergieverlust;
- Katalysator liegt im weitesten Sinne als eine "homogene Phase" im Reaktor (durchgehende Schüttung) vor, was einen Querausgleich von Druck, Temperatur und Zusammensetzung ermöglicht; dies führt zur abgeschwächten Bildung von "heißen Strömungskanälen", damit zur Unterdrückung der höchsten Temperaturen im Reaktor, den sog. "Hot Spots" und des weiteren zu einem geringeren Druckverlust im Reaktor, so daß kein Abgleichen der bei bekannten Verfahren eingesetzten, einzelnen Reaktionsrohre erforderlich ist;
- die Handhabung des Katalysators ist deutlich vereinfacht; eine Vorschaltstrecke oder ein Schutzbett ("guard-bed") ist durch einfache Befüllung des Reaktors über das oberste Plattenpaket hinaus möglich.

Die Erfindung wird im folgenden anhand von Figuren näher erläutert, die bevorzugte Ausführungsformen der Erfindung darstellen.

In den Figuren werden gleiche oder entsprechende Merkmale mit gleichen Bezugsziffern versehen.
- Fig. 1: stellt eine besonders bevorzugte Ausführungsform eines zur Durchführung des Verfahrens besonders geeigneten Reaktors im Längsschnitt dar,
- Fig. 1a: einen Querschnitt durch den Reaktor aus Fig. 1,
- Fig. 1b: einen Längsschnitt durch eine Wärmetauscherplatte des Reaktors aus Fig. 1,
- Fig. 1c: eine bevorzugte Anordnung der Schweißstellen der Wärmetauscherplatte aus Fig. 1b,
- Fig. 2: einen Längsschnitt durch einen für die Durchführung des Verfahrens besonders geeigneten Reaktor mit Gleichstromführung von Reaktionsgemisch und Wärmetauschmittel,
- Fig. 3: einen Längsschnitt durch eine weitere bevorzugte Ausführungsform eines für die Durchführung des Verfahrens besonders geeigneten Reaktors mit Gegenstromführung von Reaktionsgemisch und Wärmetauschmittel,
- Fig. 4: einen Längsschnitt durch einen für die Durchführung des Verfahrens geeigneten quaderförmigen Reaktor,
- Fig. 4a: einen vergrößerten Ausschnitt aus dem in Fig. 4 dargestellten Reaktor zur Verdeutlichung der Bauweise der Reaktorplatten,
- Fig. 4b: einen Querschnitt durch den in Fig. 4 dargestellten Reaktor und
- Fig. 5: einen Längsschnitt durch einen für die Durchführung des Verfahrens geeigneten Reaktor, der beispielhaft drei Reaktionszonen aufweist.

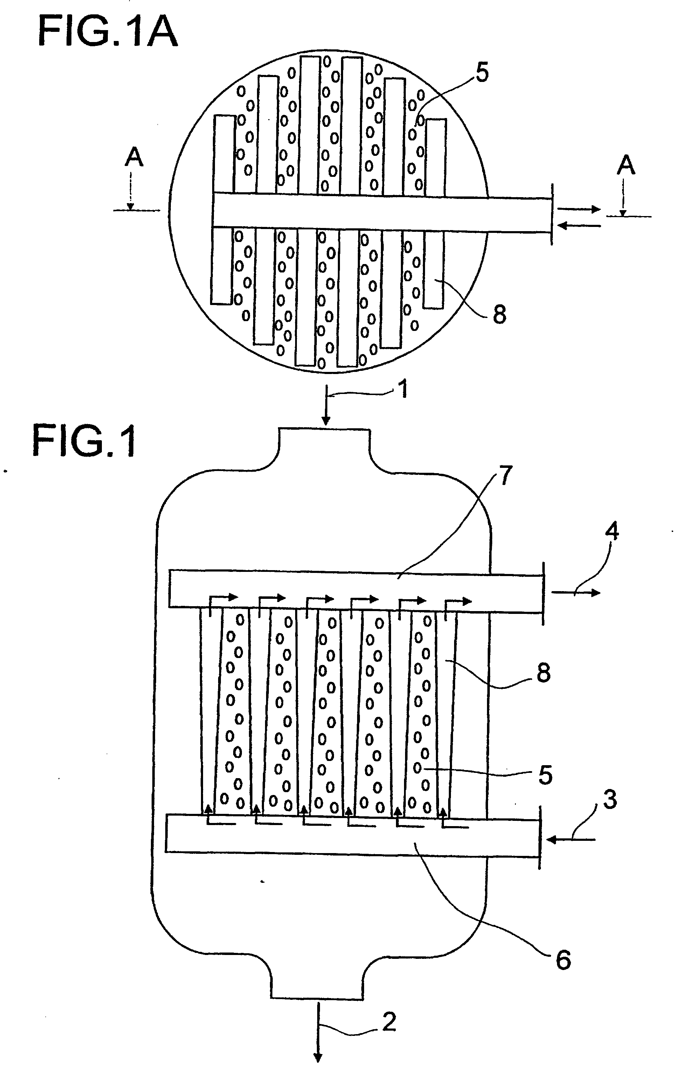

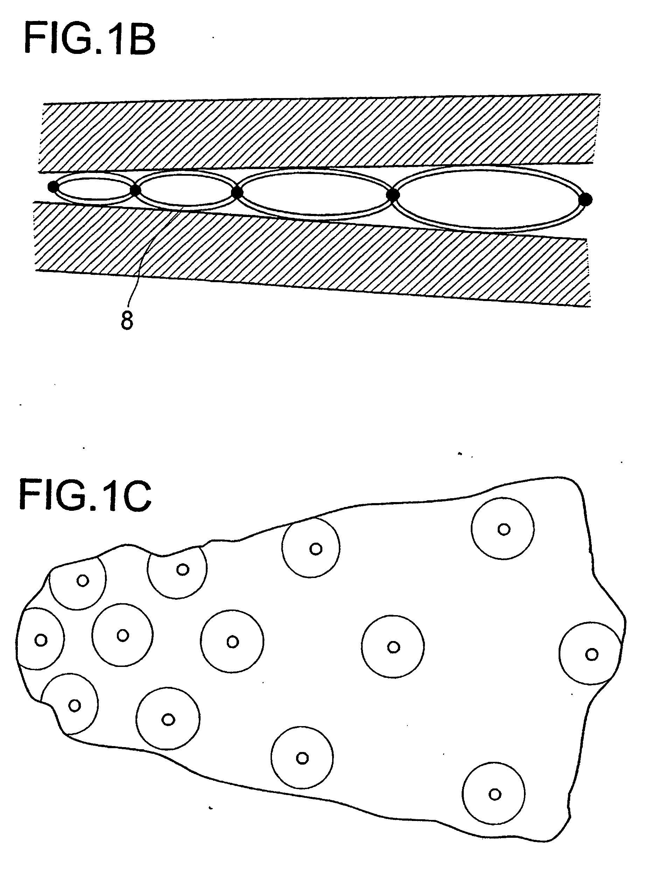

Der in Fig. 1 im Längsschnitt dargestellte Reaktor hat die Form eines Zylinders mit Zuführung des Reaktionsgemisches (1) im oberen Bereich und Abführung des Produktgemisches (2) im unteren Reaktorbereich. Das Reaktionsgemisch (1) wird über die Katalysatorschüttung (5) geführt. Im Reaktorinnenraum sind in Reaktorlängsrichtung Wärmetauscherplatten (8) angeordnet, die die keilförmige Ausbildung aufweisen. Die Reaktorplatten werden von einem Wärmetauschmittel durchströmt, das über eine Zuführung (3) und eine Verteilerleitung (6) eingebracht und über eine Sammelleitung (7) und eine Abführleitung (4) abgeführt wird. Der Querschnitt in Fig. 1a verdeutlicht die im wesentlichen parallele Anordnung der Wärmetauscherplatten (8).

Die Fig. 1b und 1c machen die keilförmige Ausbildung der Wärmetauscherplatten (8) sowie deren Ausbildung durch miteinander punktverschweißte Bleche deutlich.

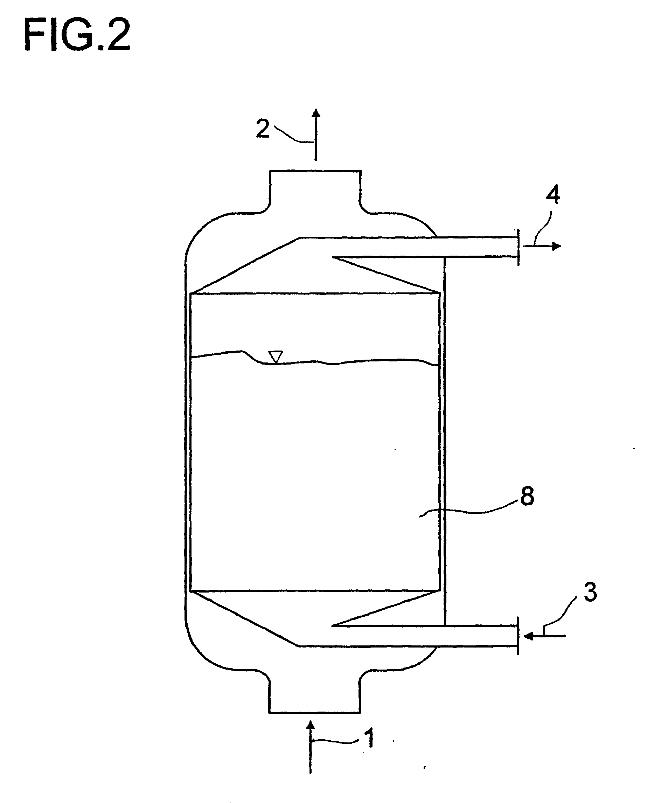

Fig. 2. zeigt beispielhaft einen Längsschnitt durch einen Reaktor mit Gleichstromführung von Reaktionsgemisch und Wärmetauschmittel. Die Fig. 2 verdeutlicht, daß in den Wärmetauscherplatten (8) der Flüssigkeitsspiegel des Wärmetauschmittels lediglich bis zu einer bestimmten Höhe steht, das Wärmetauschmittel also darüber verdampft. Die Wärmeabführung findet somit durch Siedekühlung statt.

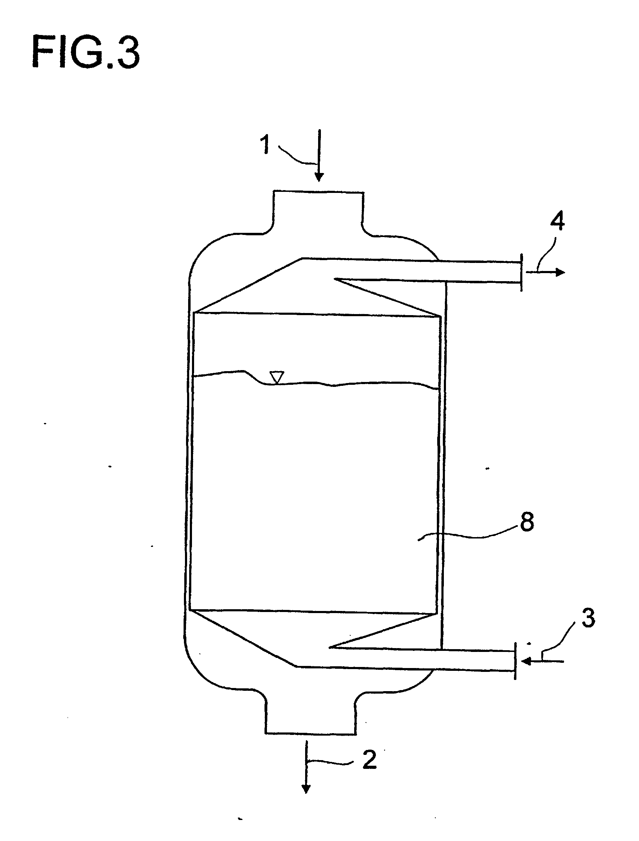

In Fig. 3 ist beispielhaft eine Gegenstromführung von Reaktionsgemisch und Wärmetauschmittel dargestellt.

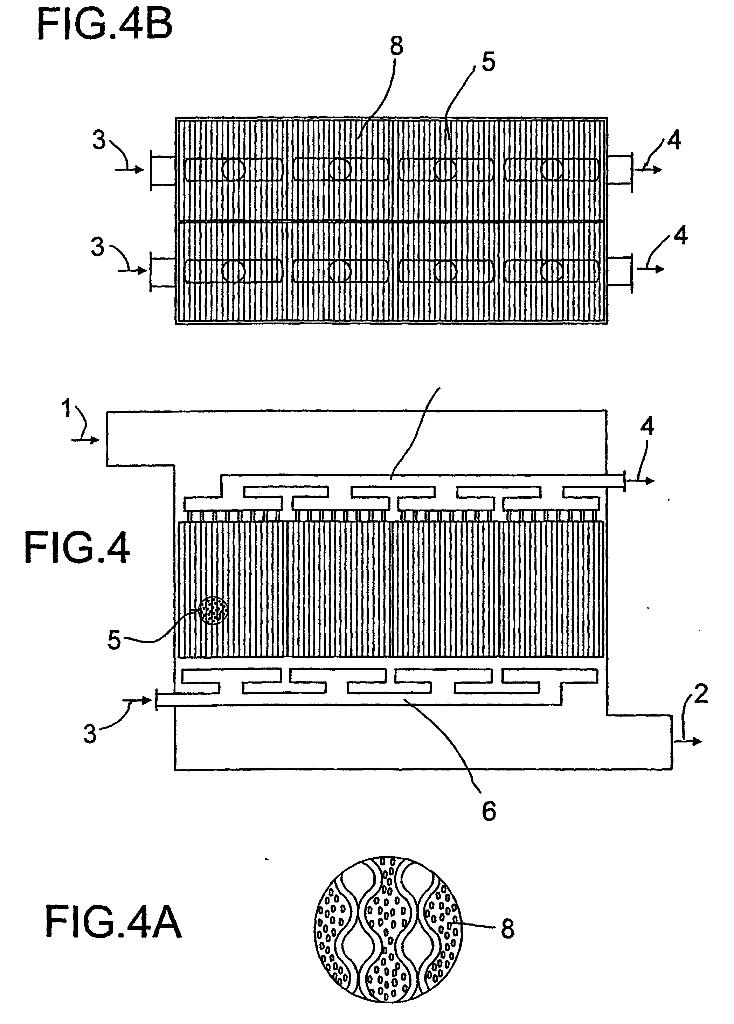

Fig. 4 zeigt einen Längsschnitt durch einen quaderförmigen Reaktor; die Ausbildung der Wärmetauscherplatten (8) ist im in Fig. 4a vergrößert dargestellten Ausschnitt verdeutlicht. Fig. 4b zeigt einen Querschnitt durch den in Fig. 4 dargestellten quaderförmigen Reaktor.

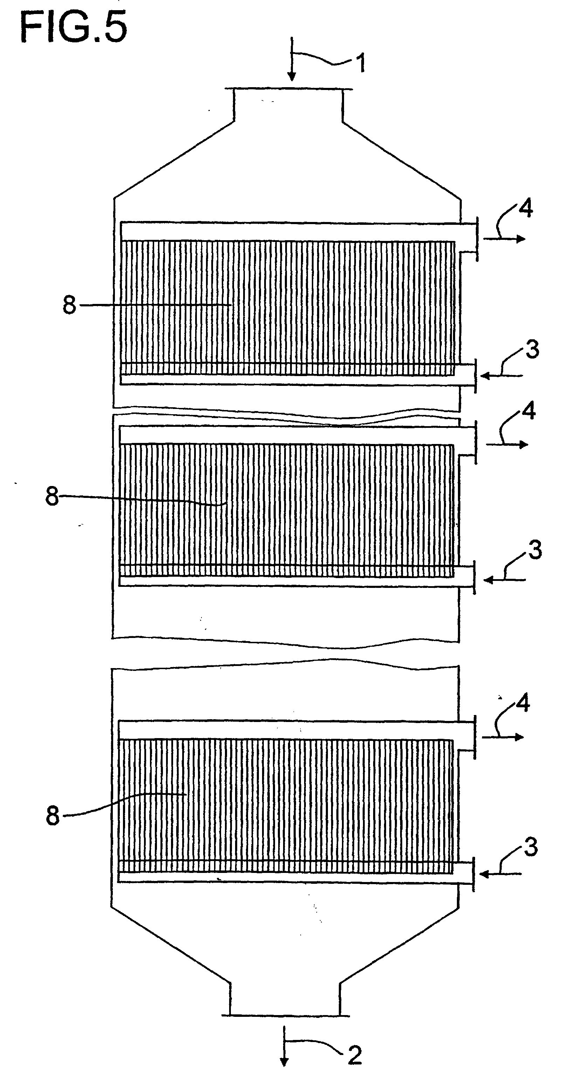

Der in Fig. 5 im Längsschnitt dargestellte Reaktor weist beispielhaft drei Reaktionszonen mit jeweils getrennten Wännetauschmittel-Kreisläufen auf.

## Patentansprüche

1. Verfahren zur Herstellung von Maleinsäureanhydrid durch heterogen katalysierte Gasphasenoxidation in einem Reaktor mit Zuführung (1) für das Reaktionsgemisch an einem Reaktorende und Abführung (2) am entgegengesetzten Reaktorende sowie im Reaktorinnenraum angeordneten Einrichtungen (8) zur Abführung der Reaktionswärme, die von einem Wärmetauschmittel durchströmt sind, **dadurch gekennzeichnet, daß** die Einrichtungen (8) Wärmetauscherplatten sind.

2. Verfahren nach Anspruch 1, **dadurch gekennzeichnet, daß** ein flüssiges Wärmetauschmittel eingesetzt wird, das beim Durchströmen der Wärmetauscherplatten (8) zumindest teilweise verdampft.

3. Verfahren nach Anspruch 1 oder 2, **dadurch gekennzeichnet, daß** die Wärmetauscherplatten aus mindestens zwei, am Umfang druckdicht verbundenen und in einem Raster von 15 bis 80 mm durch punktförmige Verbindung gegeneinander abgestützte und durch Innendruck aufgeweitete Metallbleche gebildet werden, deren so gebildeter Innenraum von dem Wärmetauschmittel durchströmt wird.

4. Verfahren nach Anspruch 3, **dadurch gekennzeichnet, daß** die Wärmetauscherplatten zu planparallelen Paketen geschichtet werden, zu Ringen oder Ringsegmenten gebogen und dann konzentrisch angeordnet werden, oder spiralförmig gebogen werden.

5. Verfahren nach Anspruch 4, **dadurch gekennzeichnet, daß** der Plattenabstand der einzelnen Plattenpakete in Strömungsrichtung entsprechend der mit zunehmendem Reaktionsfortschritt abnehmenden Leistungsdichte zunimmt.

6. Verfahren nach einem der vorhergehenden Ansprüche, **dadurch gekennzeichnet, daß** ein flüssiges anorganisches oder organisches Wärmetauschmittel oder ein gasförmiges Wärmetauschmittel eingesetzt wird.

7. Verfahren nach einem der vorhergehenden Ansprüche, **dadurch gekennzeichnet, daß** die Wärmetauscherplatten (8) parallel zueinander angeordnet sind.

8. Verfahren nach einem der vorhergehenden Ansprüche, **dadurch gekennzeichnet, daß** die dem Reaktionsgemisch zugewandten Flächen der Wärmetauscherplatten vollständig oder teilweise katalytisch beschichtet sind.

9. Verfahren nach einem der vorhergehenden Ansprüche, **dadurch gekennzeichnet, daß** das Reaktionsgemisch n-Butan, Sauerstoff und ggf. weitere Komponenten enthält.

## Claims

1. A process for the preparation of maleic anhydride by heterogeneously catalyzed gas-phase oxidation in a reactor with feed (1) for the reaction mixture at one end of the reactor and discharge (2) at the opposite end of the reactor, and with devices (8) for dissipating the heat of reaction which are arranged in the reactor interior and through which a heat-exchange medium flows, wherein the devices (8) are heat-exchanger plates.

2. A process as claimed in claim 1, wherein use is made of a liquid heat-exchange medium which evaporates at least partially on flowing through the heat-exchanger plates (8).

3. A process as claimed in claim 1 or 2, wherein the heat-exchanger plates are formed from at least two metal sheets connected in a pressure-tight manner at the periphery and supported against one another in a 15 to 80 mm grid by punctiform bonding, preferably spot welding, and expanded by internal pressure, with the heat-exchange medium flowing through their internal space formed in this way.

4. A process as claimed in claim 3, wherein the heat-exchanger plates are layered to give plane-parallel packages, bent to give rings or ring segments and then arranged concentrically, or bent in a spiral manner.

5. A process as claimed in claim 4, wherein the plate separation of the individual plate packages increases in the flow direction corresponding to the reduction in performance density with increasing reaction progress.

6. A process as claimed in one of the preceding claims, wherein a liquid inorganic or organic heat-exchange medium or a gaseous heat-exchange medium is employed.

7. A process as claimed in one of the preceding claims, wherein the heat-exchanger plates (8) are arranged parallel to one another.

8. A process as claimed in one of the preceding claims, wherein the surfaces of the heat-exchanger plates facing the reaction mixture are provided with a full or partial catalytic coating.

9. A process as claimed in one of the preceding claims, wherein the reaction mixture comprises n-butane, oxygen and, if desired, further components.

## Revendications

1. Procédé de préparation d'un anhydride maléique au moyen d'une oxydation en phase gazeuse par catalyse hétérogène dans un réacteur comprenant une alimentation (1) pour le mélange réactionnel située à une extrémité du réacteur et une évacuation (2) située à l'extrémité opposée du réacteur ainsi que des dispositifs (8) placés dans l'espace interne du réacteur destinés à dissiper la chaleur dégagée par la réaction, qui sont traversés par fluide caloporteur, **caractérisé en ce que** les dispositifs (8) sont des plaques d'échange thermique.

2. Procédé selon la revendication 1, **caractérisé en ce que** l'on met en oeuvre fluide caloporteur, qui est au moins partiellement vaporisé en traversant les plaques d'échange thermique.

3. Procédé selon la revendication 1 ou 2, **caractérisé en ce que** les plaques d'échange thermique sont composées d'au moins deux plaques métalliques fixées de façon étanche à leur périphérie, appuyées l'une contre selon un réseau de 15 à 80 mm par l'intermédiaire d'une liaison ponctuelle et s'élargissant sous l'action de la pression interne, dont l'espace interne ainsi formé est traversé par le fluide caloporteur.

4. Procédé selon la revendication 3, **caractérisé en ce que** les plaques d'échange thermique sont superposées en lots à faces planes et parallèles, courbées en cercles ou en segments de cercle et disposées dans ce cas de manière concentrique, ou courbées en forme de spirale.

5. Procédé selon la revendication 4, **caractérisé en ce que** la distance entre les plaques des lots de plaques individuels dans la direction du courant augmente proportionnellement à la puissance volumique diminuant avec la progression de la réaction.

6. Procédé selon l'une quelconque des revendications précédentes, **caractérisé en ce que** l'on met en oeuvre fluide caloporteur organique ou inorganique ou un gaz caloporteur.

7. Procédé selon l'une quelconque des revendications précédentes, **caractérisé en ce que** les plaques d'échange thermique (8) sont disposées parallèlement entre elles.

8. Procédé selon l'une quelconque des revendications précédentes, **caractérisé en ce que** les surfaces des plaques d'échange thermique en contact avec le mélange réactionnel sont recouvertes complètement ou partiellement de catalyseur.

9. Procédé selon l'une quelconque des revendications précédentes, **caractérisé en ce que** le mélange réactionnel contient du n-butane, de l'oxygène et éventuellement d'autres composants.
